(19) Europäisches Patentamt
European Patent Office
Office européen des brevets

(11) EP 0 701 997 B1

(12) **EUROPÄISCHE PATENTSCHRIFT**

(45) Veröffentlichungstag und Bekanntmachung des Hinweises auf die Patenterteilung:
**16.02.2000 Patentblatt 2000/07**

(51) Int Cl.[7]: **C07C 215/16**, C07C 213/04, C08K 5/18

(21) Anmeldenummer: **95113755.3**

(22) Anmeldetag: **01.09.1995**

(54) **Umsetzungsprodukte aus Anilinen und Bisphenolbisglycidylethern, ein Verfahren zu ihrer Herstellung und ihre Verwendung als Härtungsbeschleuniger**

Reaction products of anilines and bisphenolgylcidylethers, a process for their preparation and their use as hardening accelerators

Produits de la réaction entre des anilines et des éthers de bisphénolbisglycidyle, un procédé pour leur préparation et leur emploi comme accélérateurs de durcissement

(84) Benannte Vertragsstaaten:
**AT BE CH DE ES FR GB GR IT LI NL PT SE**

(30) Priorität: **14.09.1994 DE 4432648**

(43) Veröffentlichungstag der Anmeldung:
**20.03.1996 Patentblatt 1996/12**

(73) Patentinhaber: **BAYER AG**
**51368 Leverkusen (DE)**

(72) Erfinder:
- **Meier, Helmut-Martin, Dr.**
  **D-40883 Ratingen (DE)**
- **Fischer, Wolfgang, Dr.**
  **D-40668 Meerbusch (DE)**
- **Clemens, Horst**
  **D-47802 Krefeld (DE)**

(56) Entgegenhaltungen:
**EP-A- 0 084 784**
**EP-A- 0 510 265**
**DE-A- 2 365 257**
**GB-A- 2 120 653**
**US-A- 4 284 551**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue, N,N-disubstituierte Arylamine auf Basis von sekundären aromatischen Aminen und bestimmten Bisepoxiden, ein Verfahren zu ihrer Herstellung und ihre Verwendung als Härtungsbeschleuniger für ungesättigte Acryl- und Polyesterharze, insbesondere in kalt härtenden Spartelmassen.

Nach EP 84 784 ist es bekannt, N,N-Bis-(β-hydroxyalkyl)arylamin-Derivate als Härtungsbeschleuniger für Spachtelmassen einzusetzen. Nach BE 625 701 ist es bekannt, aromatische Amine mit Glycidylendgruppen zur Härtung von ungesättigten Polyesterharzen einzusetzen. Nach GB 2 120 653 ist es bekannnt, Umsetzungsprodukte aus primären Aminen mit Epoxiden als Härtungsbeschleuniger einzusetzen. Nach US 4 243 763 ist es bekannt, tertiäre Amine, abgeleitet aus p-Aminophenylessigsäure, als Beschleuniger für Peroxid-katalysierte Polymerisationen von ungesättigten Polyestern einzusetzen. Nach US 4 297 158 ist es bekannt, Umsetzungsprodukte aus N-Methylanilin und Epoxiden als Beschleuniger für Acrylate in Polyurethan-Elastomer-Klebstoffen einzusetzen. N-Ethyl-m-toluidin und N-Ethyl-p-toluidin sind nicht als Ausgangsstoffe eingesetzt worden. Eine Verwendung in Spachtelmasse ist nicht beschrieben. Nach US 4 284 551 ist es bekannt, tertiäre aromatische Amine, abgeleitet aus p-Aminophenethanol als Beschleuniger für Peroxid-katalysierte Polymerisationen von ungesättigten Polyesterharzen einzusetzen. Nach DE 1 544 698 sind di-tertiäre aromatische Amine als Beschleuniger für ungesättigte Polyester bekannt.

Auch das Prinzip, aromatische Amine als Beschleuniger einzusetzen, die aufgrund ihrer funktionellen Gruppen mit ungesättigten Polyestern reagieren und damit kaum aus dem gehärteten Harz migrieren können, ist bekannt. Über primäre OH-Gruppen durch Ver- oder Umesterung können Beschleuniger nach folgenden Literaturstellen in ungesättigte Polyester eingebaut werden: EP 84 784, US 4 243 763, US 4 284 551, DE 1 544 698. Über Epoxidgruppen durch Reaktion mit Säure- oder Hydroxylgruppen können Beschleuniger nach folgenden Literaturstellen in ungesättigte Polyester eingebaut werden: BE 625 701, GB 2 120 653.

Wie nachfolgende Vergleichsbeispiele belegen, ergeben sich aufgrund der Einbaubarkeit Nachteile bei der Verwendung in Spachtelmassen hinsichtlich zu geringer Reaktivität oder zu schlechter Schleifbarkeit wie auch in EP 84 784 dokumentiert. Dies gilt auch für die Beschleuniger gemäß US 4 297 158, die klebrige Oberflächen ergeben.

Die der Erfindung zugrunde liegende Aufgabe bestand nun darin, neue Beschleuniger für ungesättigte Polyesterharze zur Verfügung zu stellen, die die oben geschilderten Nachteile nicht aufweisen, d.h. insbesondere, die aus Spachtelmassen auf Basis von ungesättigten Polyesterharzen nicht migrieren und eine gute Schleifbarkeit der gehärteten Spachtelmassen gewährleisten. Diese Erfindung konnte mit der Bereitstellung der nachstehend näher beschriebenen erfindungsgemäßen Verbindungen gelöst werden.

Gegenstand der Erfindung sind Verbindungen der Formel (I)

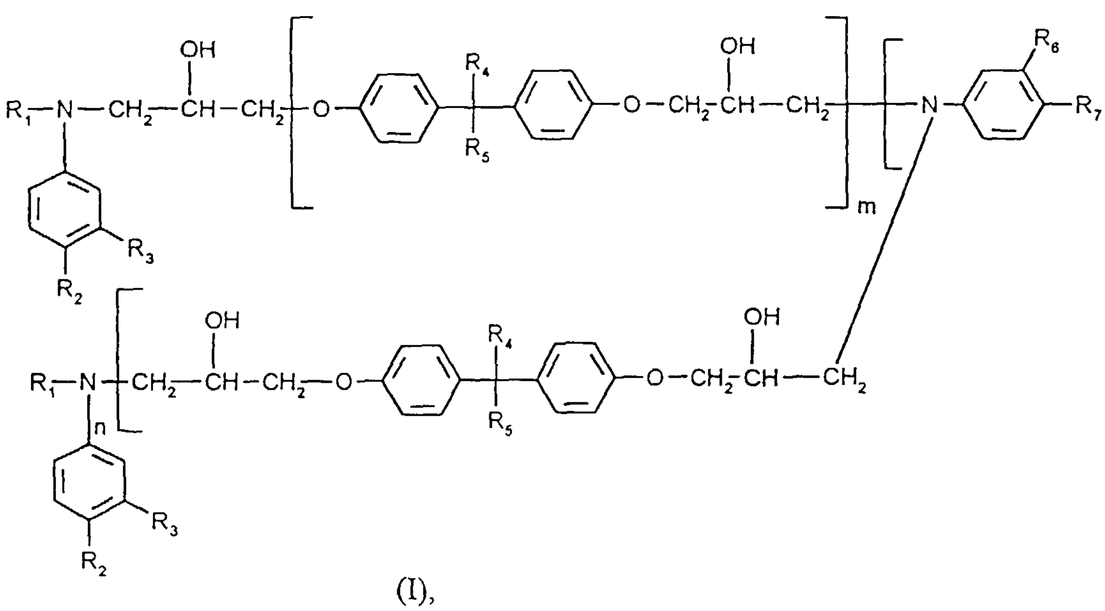

(I),

in welcher

R 1 für einen Alkyl- oder Cycloalkylrest mit 1 bis 6 Kohlenstoffatomen steht,

$R_2$ und $R_3$ für gleiche oder verschiedene Reste stehen und Wasserstoff, Alkyl-oder Cycloalkylreste mit 1 bis 6 Kohlenstoffatomen oder Halogenatome bedeuten,

$R_4$ und $R_5$ jeweils für Methylreste stehen oder zusammen mit den zwischen den aromatischen Ringen angeordneten Kohlenstoffatom einen, gegebenenfalls inerte Substituenten aufweisenden Cyclohexanrest binden,

$R_6$ und $R_7$ für gleiche oder verschiedene Reste stehen und Wasserstoff und Alkyl- oder Cycloalkylreste mit 1 bis 6 Kohlenstoffatomen oder Halogenatome bedeuten,

m für eine ganze oder (im statistischen Mittel) gebrochene Zahl von 1 bis 3 steht und

n für eine ganze oder (im statistischen Mittel) gebrochene Zahl von 0 bis 2 steht,

mit der Maßgabe, daß im Falle von n = 0, $R_1$ für einen Ethylrest steht, $R_2$ für eine Methylgruppe steht, wenn $R_3$ Wasserstoff bedeutet und $R_3$ für eine Methylgruppe steht, wenn $R_2$ Wasserstoff bedeutet.

Gegenstand der Erfindung ist auch ein Verfahren zur Herstellung der Verbindungen (I), welches dadurch gekennzeichnet ist, daß man ein Amin der Formel (II)

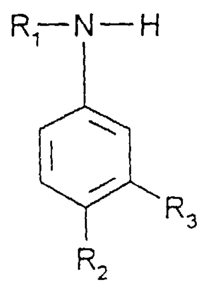

(II)

mit einem Diepoxid der Formel (III)
unter Einhaltung eines Molverhältnisses (II):(III) von 1,25:1 bis 2:1 innerhalb des Temperaturbereichs von 100 bis 250°C umsetzt, wobei $R_1$ bis $R_7$ und n die obengenannte Bedeutung haben und m für eine ganze oder (im statistischen Mittel) gebrochene Zahl von 0 bis 2 steht.

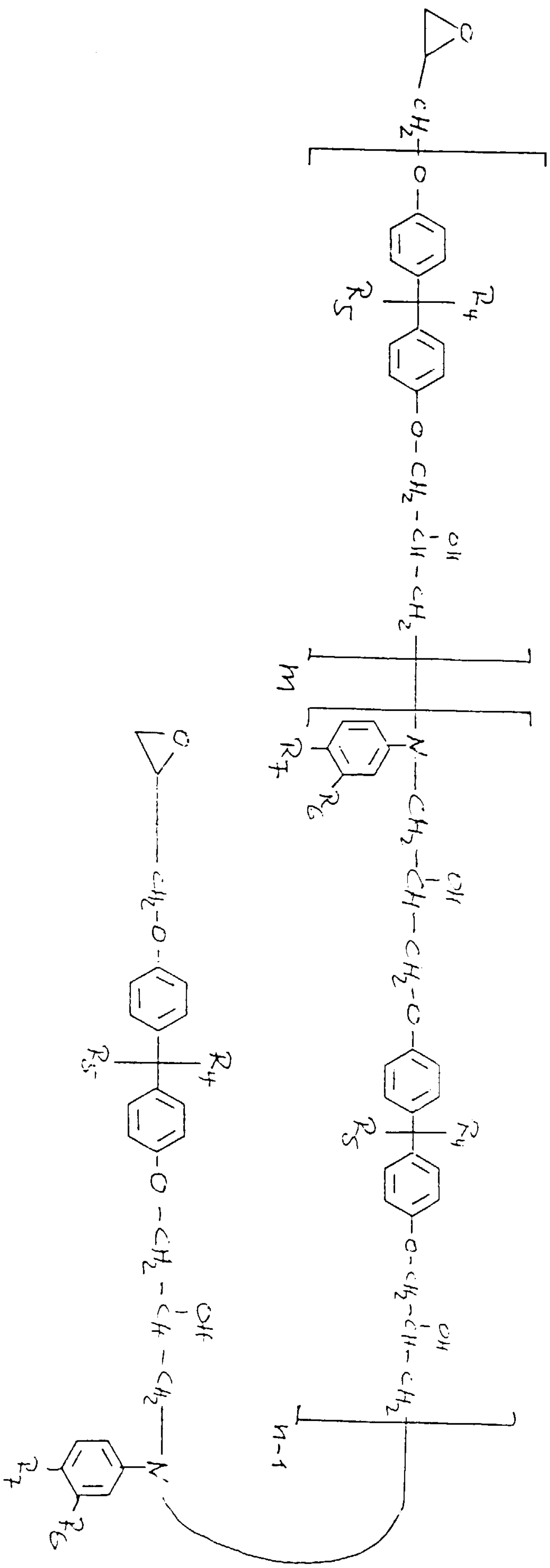

Formel (III)

Gegenstand der Erfindung ist schließlich auch die Verwendung der neuen Verbindungen (I) als Härtungsbeschleuniger für ethylenisch ungesättige kalt härtbare Acryl- und Polyesterharze.

Zur Durchführung des erfindungsgemäßen Verfahrens geeignete Amine der Formel (II) sind beliebige N-monosubstituierte Aniline, die der Formel (II) und der oben gemachten Definition der Reste $R_1$ bis $R_3$ entsprechen. Zu den bevorzugten Aminen (II) gehören jene, für welche $R_1$ für eine Methyl- und insbesondere Ethylgruppe steht und jeweils einer der Reste $R_2$ bzw. $R_3$ für eine Methylgruppe und der jeweils andere der genannten Reste für ein Wasserstoffatom stehen. Konkrete Beispiele für geeignete Amine der Formel (II) sind N-Ethyl-p-toluidin, N-Ethyl-m-toluidin, N-Ethyl-3-chlor-4-methylanilin, N-Ethyl-4-chlor-3-methylanilin, N-Ethyl-3,4-dimethylanilin, N-Ethyl-p-cyclohexylanilin, N-Ethyl-p-tert.-butylanilin. N-Ethyl-p-toluidin und N-Ethyl-m-toluidin sind besonders bevorzugt.

Für das erfindungsgemäße Verfahren geeignete Diepoxide der Formel (III) sind insbesondere Glycidylether von Bisphenol A (n = O, $R_4 = R_5 = CH_3$) mit Epoxidäquivalentgewichten von 142 bis 4000, vorzugsweise von 170 bis 1000.

Weitere, für das erfindungsgemäße Verfahren geeignete Dipoxide (III) sind solche mit eingebauten tert. Stickstoffatomen (n = 1-2). Es handelt sich hierbei um Umsetzungsprodukte von Bisphenolbisglycidylethern der soeben genannten Art mit primären Aminen der Formel (IV)

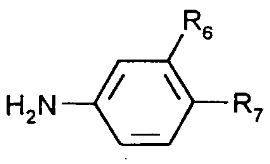

(IV),

für welche

$R_6$ und $R_7$ die bereits obengenannte Bedeutung haben und vorzugsweise für Wasserstoff oder $C_1$-$C_4$-Alkylreste stehen.

Die Herstellung von derartigen, tert. Stickstoffatome aufweisenden Diepoxiden ist beispielsweise in US 2 819 223 beschrieben.

Die Umsetzung der Amine (II) mit den Epoxiden (III) erfolgt innerhalb des Temperaturbereichs von 150 bis 250°C, vorzugsweise 150 bis 210°C und besonders bevorzugt 180 bis 210°C. Das Molverhältnis von Aminen der Formel (II) zu Diepoxiden der Formel (III) liegt hierbei 1,25:1 bis 2:1, vorzugsweise bei 1,5:1 bis 2:1.

Nach einer bevorzugten Ausführungsform wird bei der Durchführung des erfindungsgemäßen Verfahrens so vorgegangen, daß das Amin (II) in das auf die Reaktionstemperatur vorerhitzte Diepoxid (III) allmählich eingetragen wird, wobei die Geschwindigkeit der Zugabe der Geschwindigkeit der Umsetzung entspricht.

Die erfindungsgemäße Umsetzung kann durch Oxalkylierungskatalysatoren, wie z.B. tertiäre aliphatische Amine, Phosphine, Ammonium- und Phosphoniumsalze, Erdalkali- und Alkalimetalle, -hydroxide, -salze und-alkoholate beschleunigt werden.

Die auf diese Weise erhaltenen erfindungsgemäßen Verbindungen der Formel (I) stellen wertvolle Härtungsbeschleuniger für ungesättigte Acryl- und insbesondere Polyesterharze dar und werden insbesondere in kalt härtenden Spachtelmassen auf Basis dieser Harze, insbesondere auf Basis von ungesättigten Polyesterharzen eingesetzt. Zu diesem Zweck werden die Verbindungen (I) vorzugsweise in einer Konzentration entsprechend 0,008 bis 0,4, insbesondere 0,04 bis 0,2 Gew.-% tertiären Aminstickstoffes der Verbindungen (I), bezogen auf zu härtendes Harz, eingesetzt.

Die erfindungsgemäßen Verbindungen der Formel (I) stellen im allgemeinen harte, spröde Harze dar. Zur besseren Handhabung können die Harze - zweckmäßigerweise im Zuge des Herstellungsverfahrens - in geeigneten Lösungsmitteln oder Lösungsmittelgemischen gelöst werden. Bevorzugte derartige Lösungsmittel sind mit dem Polyester copolymerisierbare Monomere, wie Styrol, α-Methylstyrol, Ester der Methacrylsäure u. dgl. Außerdem können in begrenztem Umfang, der sich nach dem anwendungstechnischen Zweck richtet, polymerisationsinerte Lösungsmittel, wie z.B. n-Butylacetat, Cyclohexanon oder Ethylenglykoldimethylether, mitverwendet werden. In dieser Form lassen sich die Beschleuniger den Harzen in jeder gewünschten Konzentration leicht zudosieren.

Mit den erfindungsgemäßen Verbindungen I härtbare Harze sind im Prinzip alle ethylenisch ungesättigten Verbindungen oder Gemische, die sich in Gegenwart von Diacylperoxiden polymerisieren lassen, vorzugsweise ungesättigte Polyesterharze und Acrylharze.

"Ungesättigte Polyesterharze" in diesem Sinne sind Mischungen von 30 bis 75 Gew.-Teilen α,β-ethylenisch ungesättigter Polyester und 70 bis 25 Gew.-Teilen damit copolymerisierbarer ungesättigter Monomerer.

Derartige α,β-ethylenisch ungesättigte Polyester sind die üblichen Polykondensationsprodukte mindestens einer α,β-ethylenisch ungesättigten Dicarbonsäure mit in der Regel 4 oder 5 C-Atomen oder deren esterbildenden Derivate, gegebenenfalls in Abmischung mit bis zu 90 Mol-%, bezogen auf die ungesättigten Säurekomponenten, mindestens einer aliphatischen gesättigten Dicarbonsäure mit 4 bis 10 C-Atomen oder einer cycloaliphatischen, araliphatischen oder aromatischen Dicarbonsäure mit 8 bis 10 C-Atomen oder deren esterbildenden Derivate mit mindestens einer Polyhydroxyverbindung, insbesondere Dihydroxyverbindung, mit 2 bis 8 C-Atomen - also Polyester, wie sie z.B. bei J.R. Lawrence, "Polyester Resins", Reinbold Publ. Corp., New York 1960, S. 18 f., und im Kunststoff-Handbuch, Bd. VIII ("Polyester"), Carl Hanser Verlag, München 1973, S. 247 - 312, beschrieben sind.

Die Säurezahlen der Polyester sollen zwischen 1 und 100, vorzugsweise zwischen 55 und 70, die OH-Zahlen zwischen 10 und 150, vorzugsweise zwischen 20 und 100, und die als Zahlenmittel gemessenen Molekulargewichte $\overline{M}n$ zwischen ca. 500 und 5.000, vorzugsweise zwischen ca. 1.000 und 3.000 liegen (dampfdruckosmometrisch gemessen in Dioxan und Aceton; bei differierenden Werten wird der niedrigere als der korrekte angesehen).

Das bevorzugteste copolymerisierbare Monomere ist Styrol.

"Acrylharze" im Sinne der Erfindung sind (Meth-)Acryloyloxygruppen enthaltende Polyester, Polyurethane, Polyepoxide, Polyole, Polyetherpolyole, wie sie beispielsweise in den DE-OS 2 053 683, 2 261 612, 2 423 354, 2 838 691 (Polyester(meth)acrylate), in den DE-OS 1 447 929, 1 916 499, 2 115 373, in den US-PS 2 958 704, 3 297 745, in der GB-PS 743 514 (Urethan(meth)acrylate), in den DE-OS 1 921 869, 2 349 979, 2 411 760, 2 429 527, in der GB-PS 1 006 587, in den US-PS 3 066 112, 3 804 735 (Epoxy(meth)acrylate), in der DE-OS 1 770 825, in den US-PS 2 101 107, 2 413 973, 3 368 900, 3 552 986, 3 558 387 (Polyol-(meth)acrylate), in den DE-OS 2 651 507, 2 853 921 und in den US-PS 2 951 758, 3 380 831 (Polyetherpolyol(meth)acrylate) beschrieben sind.

Die vorgenannten "Acrylharze" können zur Viskositätserniedrigung, Reaktivitätserhöhung oder zur Erzielung spezieller Eigenschaften auch mit copolymerisierbaren olefinisch ungesättigten Monomeren, z.B. mit (Meth-)Acrylsäureestern einwertiger Alkohole, Hydroxyalkyl(meth)acrylaten, (Meth-)Acrylamiden, Styrol, α-Methylstyrol, durch Alkylgruppen kernsubstituierte Styrole, Divinylbenzol, (Meth-)Acrylnitril, Vinylchlorid, Vinylidenchlorid, Vinylethern, Vinylacetat oder deren Mischungen abgemischt werden. Natürlich ist es auch möglich, mindestens ein α,β-monoolefinisch ungesättigtes Monomeres, beispielsweise der vorstehend angegebenen Art, in Gegenwart der erfindungsgemäßen Verbindungen I zu polymerisieren.

Vor der Härtung werden den Harzen Polymerisationsinitiatoren, vorzugsweise Diacylperoxide oder Percarbonate, in Mengen von 1 bis 10 Gew.-%, bezogen auf zu polymerisiserendes Harz, zugesetzt. Bevorzugte Initiatoren sind z.B. Diacetylperoxid, Dibenzoylperoxid, Di-p-chlorbenzoylperoxid, Phthaloylperoxid, Succinylperoxid, Dilauroylperoxid, Acetylcyclohexansulfonylperoxid, Isopropylpercarbonat, Cyclohexylpercarbonat und Bis-(4-tert.-butylcyclohexyl)-percarbonat.

Die zu polymerisierenden Harzmassen können die üblichen Füllstoffe, Pigmente, Stabilisatoren und auch Desinfektionsmittel enthalten. Bei Verwendung auf dem Dentalgebiet kommen sowohl organische Füllstoffe, wie pulverisierte Polyacrylate, als auch anorganische Füllstoffe, wie pulverisiertes Quarz oder Glas bzw. Siliciumdioxid oder Aluminiumoxidpulver in Frage.

Erfindungsgemäß lassen sich alle Arten von Formteilen in der Kälte aushärten und auf den verschiedensten Gebieten der Bauindustrie, der Elektroindustrie, des Bootsbaus und in der Kraftfahrzeugindustrie einsetzen.

Zur erfindungsgemäß bevorzugten Herstellung von Spachtelmassen auf Basis von ungesättigten Polyesterharzen der beispielhaft genannten Art werden den Polyesterharzen 50 bis 350 Gew.-Teile, bezogen auf 100 Gew.-Teile Polyesterharz, Füllstoffe, wie Kreide, Talkum, Baryt, Aerosil, zugesetzt. Farbstoffe oder Pigmente können natürlich ebenfalls zugesetzt werden. Das Mischen der verschiedenen Komponenten der erfindungsgemäßen Formmassen erfolgt zweckmäßig in Knetern, Dissolvern oder auf Walzenstühlen.

Die Spachtelmassen eignen sich z.B. zur Ausbesserung von Karosserieblechen, Kunststoffplatten und Steinplatten jeglicher Art. Bevorzugten Einsatz finden erfindungsgemäß beschleunigte Spachtelmassen auf dem Autoreparatursektor.

Die in den nachfolgenden Beispielen genannten Teile sind Gewichtsteile; Prozentangaben beziehen sich auf das Gewicht.

## I. Herstellung von erfindungsgemäßen Aminbeschleunigern

**Beispiel 1** (Beschleuniger 1)

225 g eines Diglycidylethers des Bisphenol A mit einem Epoxidäquivalentgewicht von 500 und einem Molekulargewicht von 900 werden unter Stickstoff in 30 Minuten auf 180°C erhitzt. In 4 Stunden wird 64,9 g N-Ethyl-m-toluidin zugetropft, die Temperatur auf 200°C erhöht und noch 4 Stunden bei 200°C nachgerührt. Nach Abkühlen auf 110°C kann mit Styrol auf einen Festgehalt von 65 % eingestellt werden.
tert. Amin-Stickstoff: 2,52 % (100%iges Harz)

**Beispiel 2** (Beschleuniger 2)

200 g eines Diglycidylethers des Bisphenol A mit einem Epoxidäquivalentgewicht von 380 und einem Molekulargewicht von 770 werden unter Stickstoff in 30 Minuten auf 180°C erhitzt. In 4 Stunden wird 64,9 g N-Ethyl-m-toluidin zugetropft, die Temperatur auf 200°C erhöht und noch 4 Stunden bei 200°C nachgerührt. Nach Abkühlen auf 110°C kann mit Styrol auf einen Festgehalt von 65 % eingestellt werden.
tert. Amin-Stickstoff: 2,77 % (100%iges Harz)

**Beispiel 3** (Beschleuniger 3)

225 g eines Diglycidylethers des Bisphenol A mit einem Epoxidäquivalentgewicht von 500 und einem Molekulargewicht von 900 werden unter Stickstoff in 30 Minuten auf 180°C erhitzt. In 4 Stunden wird 64,9 g N-Ethyl-m-toluidin zugetropft, die Temperatur auf 200°C erhöht und noch 4 Stunden bei 200°C nachgerührt. Nach Abkühlen auf 110°C kann mit Styrol auf einen Festgehalt von 65 % eingestellt werden.
tert. Amin-Stickstoff: 2,39 % (100%iges Harz)

**Beispiel 4** (Beschleuniger 4)

180 g eines Diglycidylethers des Bisphenol A mit einem Epoxidäquivalentgewicht von 190 und einem Molekulargewicht von 380 werden unter Stickstoff in 30 Minuten auf 180°C erhitzt. In 4 Stunden wird 64,9 g N-Ethyl-m-toluidin zugetropft, die Temperatur auf 200°C erhöht und noch 4 Stunden bei 200°C nachgerührt. Nach Abkühlen auf 110°C kann mit Styrol auf einen Festgehalt von 65 % eingestellt werden.
tert. Amin-Stickstoff: 2,97 % (100%iges Harz)

**Beispiel 5** (Beschleuniger 5)

186,5 g eines Diglycidylethers des Bisphenol A mit einem Epoxidäquivalentgewicht von 190 und einem Molekulargewicht von 400 werden unter Stickstoff in 30 Minuten auf 160°C erhitzt. In 4 Stunden wird 23,25 g Anilin zugetropft und dann in 2 Stunden 67,5 g N-Ethyl-m-toluidin bei 160°C zugetropft, und noch 2 Stunden bei 160°C nachgerührt. Nach Abkühlen auf 110°C kann mit Styrol auf einen Festgehalt von 65 % eingestellt werden.
tert. Amin-Stickstoff: 4,26 bis 4,28 % (100%iges Harz)

**Beispiel 6** (Beschleuniger 6)

186,5 g eines Diglycidylethers des Bisphenol A mit einem Epoxidäquivalentgewicht von 190 und einem Molekulargewicht von 400 werden unter Stickstoff in 30 Minuten auf 160°C erhitzt. In 4 Stunden wird 26,75 g m-Toluidin zugetropft und dann in 2 Stunden 67,5 g N-Ethyl-m-toluidin bei 160°C zugetropft, und noch 2 Stunden bei 160°C nachgerührt. Nach Abkühlen auf 110°C kann mit Styrol auf einen Festgehalt von 65 % eingestellt werden.
tert. Amin-Stickstoff: 3,95 bis 3,98 % (100%iges Harz)

**II. Herstellung von Aminbeschleunigern zum Vergleich**

**Vergleichsbeispiel 1** (Beschleuniger nach EP 84784)

214 g m-Toluidin werden unter $N_2$ auf 195°C erhitzt und 400 g eines Diglycidylether des Bisphenol A (Epoxidäquivalent 190, Molgewicht 380) in 5 Stunden unter Rühren zugetropft. Nach 5 Stunden Nachrühren bei 180 bis 200°C wird auf 150°C abgekühlt. Nun werden innerhalb von 10 Stunden 86 g Ethylenoxid eingeleitet. Es resultiert ein sprödes Harz.
tert. Amin-Stickstoff: 3,8 %

**Vergleichsbeispiel 2** (Beschleuniger nach BE 625701)

Aus 4,8 mol m-Toluidin und 2 mol Epichlorhydrin werden 1 mol N,N-Bisglycidyl-m-toluidin hergestellt. Der Überschuß an m-Toluidin wird zur Erzeugung des Oxiranringes benötigt und nach Beendigung der Reaktion als Hydrochlorid abfiltriert.
tert. Amin-Stickstoff: 6,3 %

**Vergleichsbeispiel 3** (Beschleuniger nach GB 2120653)

71,3 g p-Toluidin (0,67 mol) werden unter $N_2$ in einem 250 ml Reaktionsgefäß vorgelegt. Man erhitzt auf 160°C und gibt, ohne weiter zu erhitzen, 180 g eines Bisphenol-A-diglycidylethers mit einem Molekulargewicht von 380 innerhalb von 15 Minuten so zu, daß die Reaktionstemperatur 170°C nicht übersteigt. 50 g Phenylglycidylether (0,33 mol) wird dann in 10 Minuten zugegeben und bei 170°C 60 Minuten gehalten.
tert. Amin-Stickstoff: 3 %

**Vergleichsbeispiel 4** (Beschleuniger nach US 4243763)

Es wird ein polymeres Amin mit 1 bis 8 Amin-Einheiten durch Addition von 380 g Diglycidylether des Bisphenol A an n-Butyl-p-aminophenylacetat hergestellt. Dieses Produkt wird dann mit einem Überschuß Methacrylsäure (2,5 mol) in Gegenwart von Butylhydroxytoluol zu einem Kondensationsprodukt umgesetzt.
tert. Amin-Stickstoff: 1,8 %

**Vergleichsbeispiel 5** (Beschleuniger nach US 4297158)

107 N-Methylanilin und 500 g des Diglycidylethers des Bisphenols A gemäß Beispiel 1 werden unter $N_2$ in einen 1 Literkolben mit Rührer, Rückflußkühler, Thermometer gefüllt. Nach Zugabe von 100 ppm Hydrochinon wird auf 100°C für 24 Stunden erhitzt.
tert. Amin-Stickstoff: 2,3 %

**Vergleichsbeispiel 6** (Beschleuniger nach US 4284551)

1 mol p-Aminophenylethanol wird mit 2 Mol eines Bisglycidylethers des Bisphenol A (Molgewicht 380, Epoxidäquivalentgewicht 180 g/val) bei 170°C 4 Stunden unter $N_2$ umgesetzt, und das Reaktionsprodukt wird dann mit 2 mol N-Ethyl-p-aminophenylethanol in 2 Stunden bei 170°C zum Vergleichsbeschleuniger abreagiert.
tert. Amin-Stickstoff: 3,4 %

**Vergleichsbeispiel 7** (Beschleuniger nach DOS 1544698)

30,2 g (N-β-hydroxyethyl)-p-toluidin, 19,7 g Ethylenbromid und 16,8 g Natriumbicarbonat werden in 100 ml Wasser 40 Stunden am Rückfluß erhitzt. Die organische Phase wird abgetrennt, im Vakuum von Resten von Ausgangsmaterial befreit und zweimal mit Wasser aus Ethanol umgefällt. Es werden 19 g Bis-((N-β-hydroxyethyl)-p-toluidino)-ethan-1,2 erhalten. tert. Amin-Stickstoff: 8 %

**III. Herstellung eines ungesättigten Polyesterharzes**

Aus 479 g (4,52 mol) Diethylenglykol und 421 g (4,3 mol) Maleinsäureanhydrid wird durch Schmelzkondensation ein Polyester hergestellt. Dabei werden gleichzeitig 177 g (1,34 mol) Dicyclopentadien addiert. Anschließend wird das Harz zu einer 65%igen Lösung in Styrol gelöst und mit 0,02 % Toluhydrochinon stabilisiert. Das erhaltene Polyesterharz hat eine Viskosität von 500 bis 650mPas (gemessen bei 23°C) und eine Säurezahl von 10.

**IV. Bestimmung der Reaktivität**

Unter Verwendung der vorgenannten Beschleuniger werden aus dem Polyesterharz kalthärtende Gießharzmassen hergestellt. Der bei den angegebenen Konzentrationen gegebene Gehalt an Amin-Stickstoff in dem kalthärtenden Harz ermöglicht einen Vergleich der Reaktivität der einzelnen Beschleuniger. Die Reaktivität wird bestimmt, indem die Beschleuniger/Harzmischung bei einer Anfangstemperatur von 25°C mit 2 % handelsüblicher Benzoylperoxid-Paste (50 % Benzoylperoxid-Gehalt) ausgehärtet wird.
Die Bestimmung von Gelzeit, Härtezeit und Maximaltemperatur erfolgt gemäß DIN 16 945.

Erfindungsgemäße Aminbeschleuniger

Die erfindungsgemäßen Beschleuniger werden als 65%ige styrolische Lösung eingesetzt.

| Beispiel | 1 | 2 | 3 | 4 | 5 | 6 |
|---|---|---|---|---|---|---|
| Beschleuniger-Zusatz (%) | 7,4 | 8,15 | 7,7 | 6,22 | 4,3 | 4,7 |
| Gesamt N (%) | 0,12 | 0,146 | 0,12 | 0,12 | 0,12 | 0,12 |
| Gelzeit (min) | 6,2 | 5,3 | 6,2 | 5,8 | 9,4 | 6,9 |
| Härtezeit (min) | 8,2 | 7,5 | 8,5 | 8,1 | 13,1 | 9,2 |
| T max (°C) | 94 | 98 | 94 | 98 | 90 | 97 |

Aminbeschleuniger nach Stand der Technik

Die Aminbeschleuniger nach Stand der Technik werden als 65%ige styrolische Lösung eingesetzt.

| Vergleichsbeispiel | 1 | 2 | 3 | 4 | 5 | 6 | 7 |
|---|---|---|---|---|---|---|---|
| Beschleuniger-Zusatz (%) | 5,0 | 3 | 6 | 10,3 | 8 | 5,5 | 2,3 |
| Gesamt N (%) | 0,12 | 0,12 | 0,12 | 0,12 | 0,12 | 0,12 | 0,12 |
| Gelzeit (min) | 7,3 | 10,1 | 8,3 | 13,3 | 9,4 | 8,7 | 1,3 |
| Härtezeit (min) | 9,4 | 14 | 10,7 | 17,2 | 14,6 | 12,3 | 2,4 |
| T max (°C) | 90 | 85 | 91 | 86 | 83 | 90 | 91 |

**V. Herstellung von Spachtelmassen**

Jeweils 100 Teile von Polyesterharz werden mit dem jeweiligen Beschleuniger, 130 Teilen Talkum, 60 Teilen Schwerspat und 7 Teilen Titandioxid (Rutil) homogenisiert.

100 g Spachtelmasse werden mit ca. 2 g einer handelsüblichen 50%igen Benzoylperoxidpaste verrührt. Die Masse wird dann in ca. 1 mm Schichtdicke auf entfettete und geschliffene Stahlblechplatten aufgetragen und die Schleifbarkeit nach bestimmten Zeiten beurteilt.

Zusammensetzung der erfindungsgemäßen Spachtelmassen

| Spachtelmasse | A | B | C | D | E | F |
|---|---|---|---|---|---|---|
| Polyesterharz Zusatz (Gew.-Teile) | 100 | 100 | 100 | 100 | 100 | 100 |
| Beschleuniger<br>Zusatz (Gew.-Teile) | 1<br>7,4 | 2<br>8,15 | 3<br>7,7 | 4<br>6,22 | 5<br>4,3 | 6<br>4,7 |
| Füllstoffe (Teile) | 197 | 197 | 197 | 197 | 197 | 197 |

Zusammensetzung der Spachtelmassen nach Stand der Technik

| Spachtelmasse | H | I | J | K | L | M | N |
|---|---|---|---|---|---|---|---|
| Polyesterharz Zusatz (Gew.-Teile) | 100 | 100 | 100 | 100 | 100 | 100 | 100 |
| Vergleichsbsp. Zusatz (Gew.-Teile) | 1<br>5,0 | 2<br>3,0 | 3<br>6,0 | 4<br>10,3 | 5<br>8,0 | 6<br>5,5 | 7<br>2,3 |
| Füllstoffe (Teile) | 197 | 197 | 197 | 197 | 197 | 197 | 197 |

**VI. Bestimmung der Schleifbarkeit**

Die Schleifbarkeit wird nach der Peroxid-Zugabe nach unterschiedlichen Zeiten bestimmt, indem die ausgehärtete Spachtelmasse mit Schleifpapier mittlerer Körnung (80er Papier) von Hand ein und derselben Person geschliffen wird. Dabei wird sowohl die Menge an abgetragenem Spachtel als auch der Grad der Zusetzung des Schleifpapiers beurteilt.

Verhalten des Spachtels beim Schleifen

1 sehr gut schleifbar
2 gut schleifbar
3 mäßig schleifbar
4 schlecht schleifbar
5 nicht schleifbar

Verhalten des Schleifpapiers beim Schleifen

a kein Zusetzen
b etwas Zusetzen
c etwas mehr Zusetzen
d stark Zusetzen
e verschmiert beim Schleifen

Schleifbarkeit der Spachtelmassen mit erfindungsgemäßen Beschleunigern

| Spachtelmasse | | A | B | C | D | E | F |
|---|---|---|---|---|---|---|---|
| Beschleuniger | | 1 | 2 | 3 | 4 | 5 | 6 |
| Shore D | (12 min) | 36 | 35 | - | 39 | - | - |
| | (1 Std) | 77 | 77 | 5 | 77 | 70 | 75 |
| Schleifbarkeit | | | | | | | |
| 12 min | | 1-2c-d | 1-2b-c | 2d | 1-2a | 2d | 2d |
| 15 min | | 1-2b | 1-2a | 1-2a-b | 1-2a | 2c | 1-2c |
| 20 min | | 1-2a | 1-2a | 1-2a | 1-2a | 1b | 1-2a |

Schleifbarkeit der Spachtelmassen mit Aminbeschleunigern nach dem Stand der Technik

| Spachtelmasse | | H | I | J | K | L | M | N |
|---|---|---|---|---|---|---|---|---|
| Vergleichsbsp. | | 1 | 2 | 3 | 4 | 5 | 6 | 7 |
| Shore D | (12 min) | 32 | - | 34 | - | - | 32 | 35 |
| | (1 Std.) | 68 | 60 | 70 | 65 | 60 | 71 | 73 |
| Schleifbarkeit | | | | | | | | |
| 12 min | | - | - | 3d | - | - | 3d | 4b |
| 15 min | | 2c | 2d | 2c | 3d | - | 3c | 4c |
| 20 min | | 2b | 2c | 2b | 2c | 4d | 2b | 3c |

## Patentansprüche

1. Verbindungen der Formel (I)

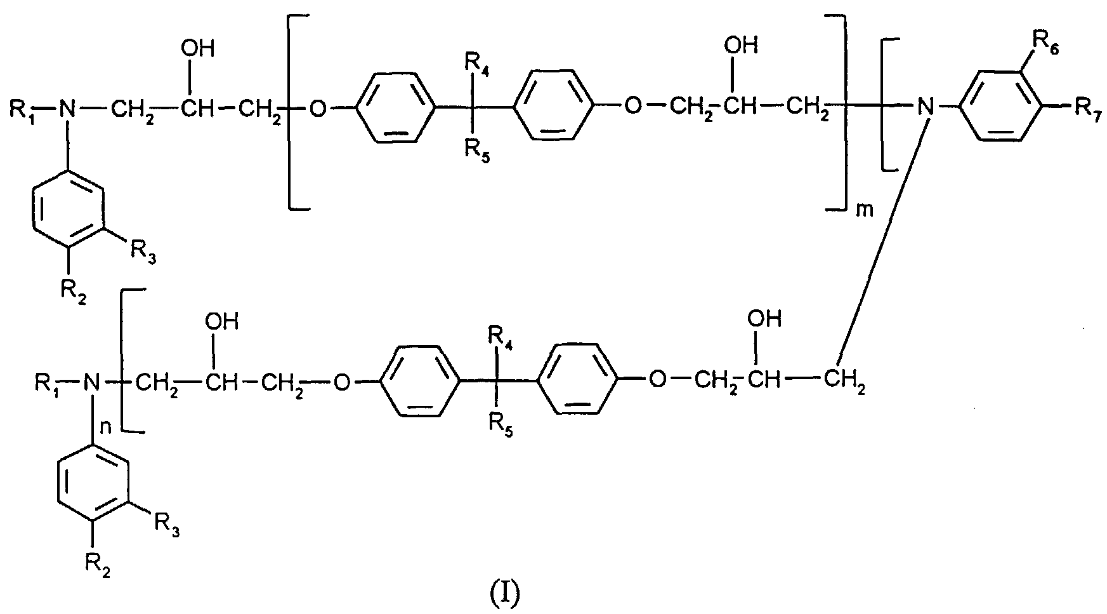

(I)

in welcher

| | |
|---|---|
| $R_1$ | für einen Alkyl-oder Cycloalkylrest mit 1 bis 6 Kohlenstoffatomen steht, |
| $R_2$ und $R_3$ | für gleiche oder verschiedene Reste stehen und Wasserstoff, Alkyl- oder Cycloalkylreste mit 1 bis 6 Kohlenstoffatomen oder Halogenatome bedeuten, |
| $R_4$ und $R_5$ | jeweils für Methylreste stehen oder zusammen mit den zwischen den aromatischen Ringen angeordneten Kohlenstoffatom einen, gegebenenfalls inerte Substituenten aufweisenden Cyclohexanrest binden, |
| $R_6$ und $R_7$ | für gleiche oder verschiedene Reste stehen und Wasserstoff Alkyl- oder Cycloalkylreste mit 1 bis 6 Kohlenstoffatomen oder Halogenatome bedeuten, |
| m | für eine ganze oder (im statistischen Mittel) gebrochene Zahl von 1 bis 3 steht und |
| n | für eine ganze oder (im statistischen Mittel) gebrochene Zahl von 0 bis 2 steht, |

mit der Maßgabe, daß im Falle von n = 0, $R_1$ für einen Ethylrest steht, $R_2$ für eine Methylgruppe steht, wenn $R_3$ Wasserstoff bedeutet und $R_3$ für eine Methylgruppe steht, wenn $R_2$ Wasserstoff bedeutet.

2. Verbindungen gemäß Anspruch 1, dadurch gekennzeichnet, daß eine der Reste $R_2$ und $R_3$ für Wasserstoff und der jeweils andere der genannten Reste für eine Methylgruppe steht.

3. Verbindungen gemäß Anspruch 1 und 2, dadurch gekennzeichnet, daß $R_1$ für eine Methyl- oder Ethylgruppe steht.

4. Verfahren zur Herstellung von Verbindungen der Formel (I), dadurch gekennzeichnet, daß man ein Amin der Formel (II)

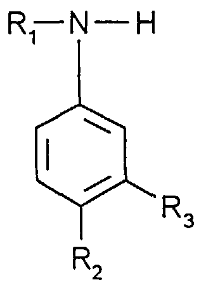

(II)

mit einem Diepoxid der Formel (III)

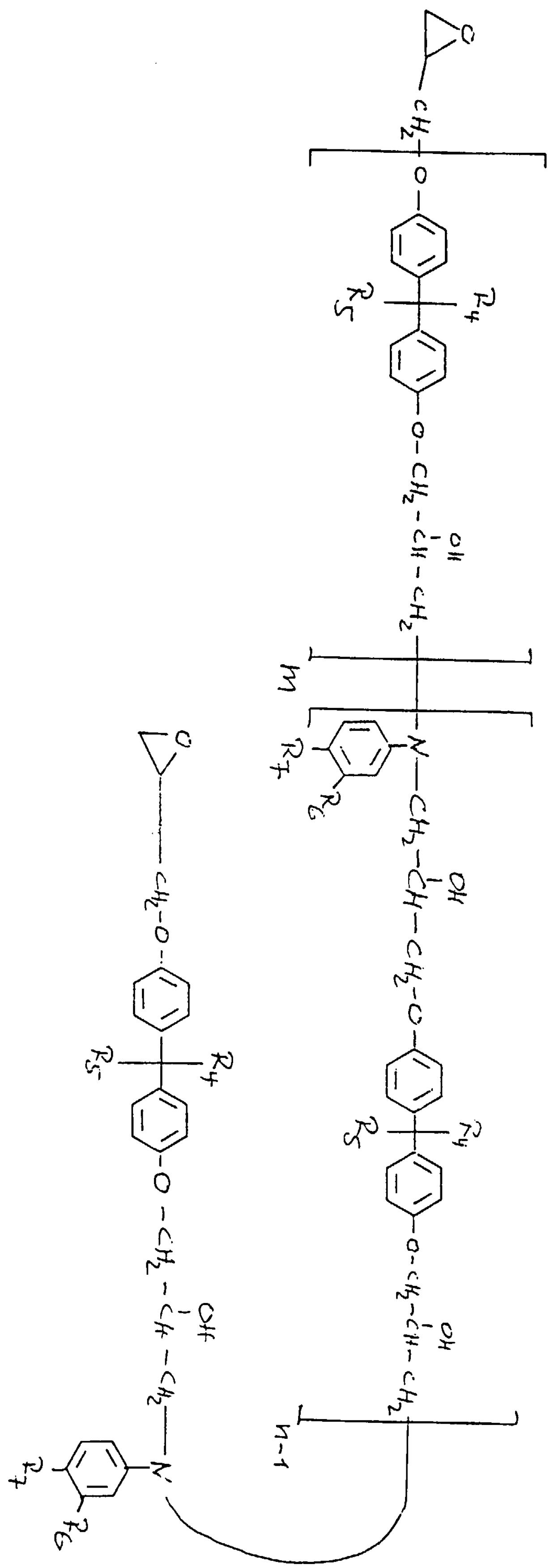

Formel (III)

unter Einhaltung eines Molverhältnisses (II):(III) von 1,25:1 bis 2:1 innerhalb des Temperaturbereichs von 100 bis 250°C umsetzt, wobei $R_1$ bis $R_7$ und m die in Anspruch 1 genannte Bedeutung haben.

**5.** Verwendung der Verbindungen gemäß Anspruch 1 bis 3 als Härtungsbeschleuniger für ethylenisch ungesättige kalt härtbare Acryl- und Polyesterharze.

**Claims**

**1.** Compounds of the formula (I)

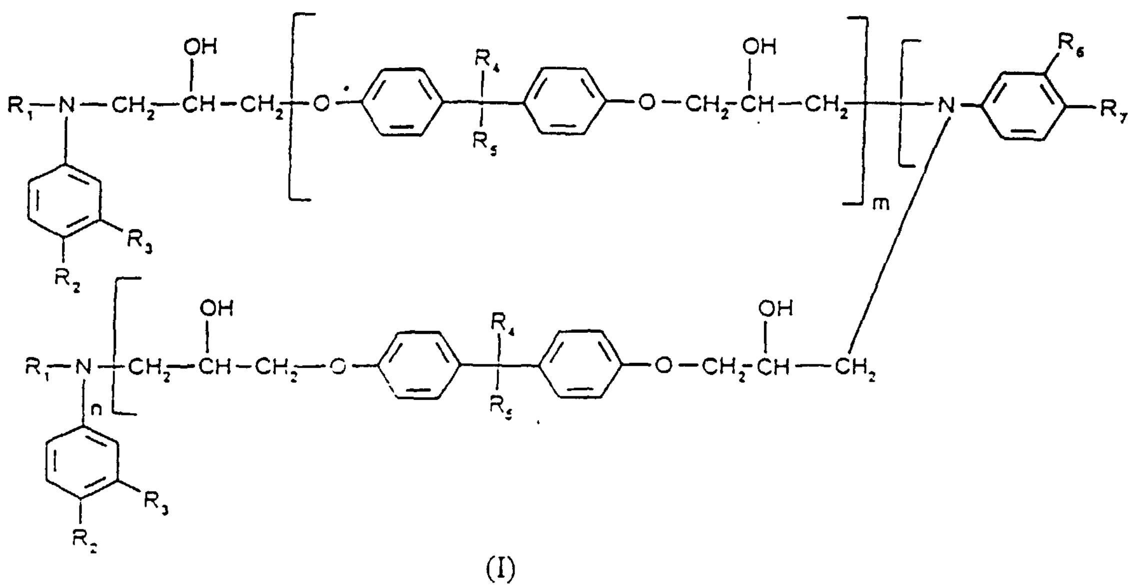

(I)

in which,

| | |
|---|---|
| $R_1$ | represents an alkyl or cycloalkyl group with 1 to 6 carbon atoms, |
| $R_2$ and $R_3$ | are identical or different groups and represent hydrogen, alkyl or cycloalkyl groups with 1 to 6 carbon atoms or halogen atoms, |
| $R_4$ and $R_5$ | each represent methyl groups or, together with the carbon atom located between the aromatic rings, form a cyclohexane group, optionally with inert substituents, |
| $R_6$ and $R_7$ | are identical or different and represent hydrogen, alkyl or cycloalkyl groups with 1 to 6 carbon atoms or halogen atoms, |
| m | represents an integer or (as a statistical average) a fractional number from 1 to 3 and |
| n | represents an integer or (as a statistical average) a fractional number from 0 to 2, |

with the proviso that in the event that n = 0, $R_1$ represents an ethyl group, $R_2$ represents a methyl group when $R_3$ is hydrogen and $R_3$ represents a methyl group when $R_2$ is hydrogen.

**2.** Compounds according to Claim 1, characterised in that one of the groups $R_2$ and $R_3$ represents hydrogen and of the other group mentioned represents a methyl group.

**3.** Compounds according to Claims 1 and 2, characterised in that $R_1$ represents a methyl or ethyl group.

**4.** A process for preparing compounds of the formula (I), characterised in that an amine of the formula (II)

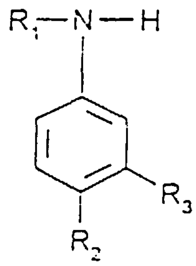

(II)

reacts with a diepoxide of the formula (III)

Formula (III)

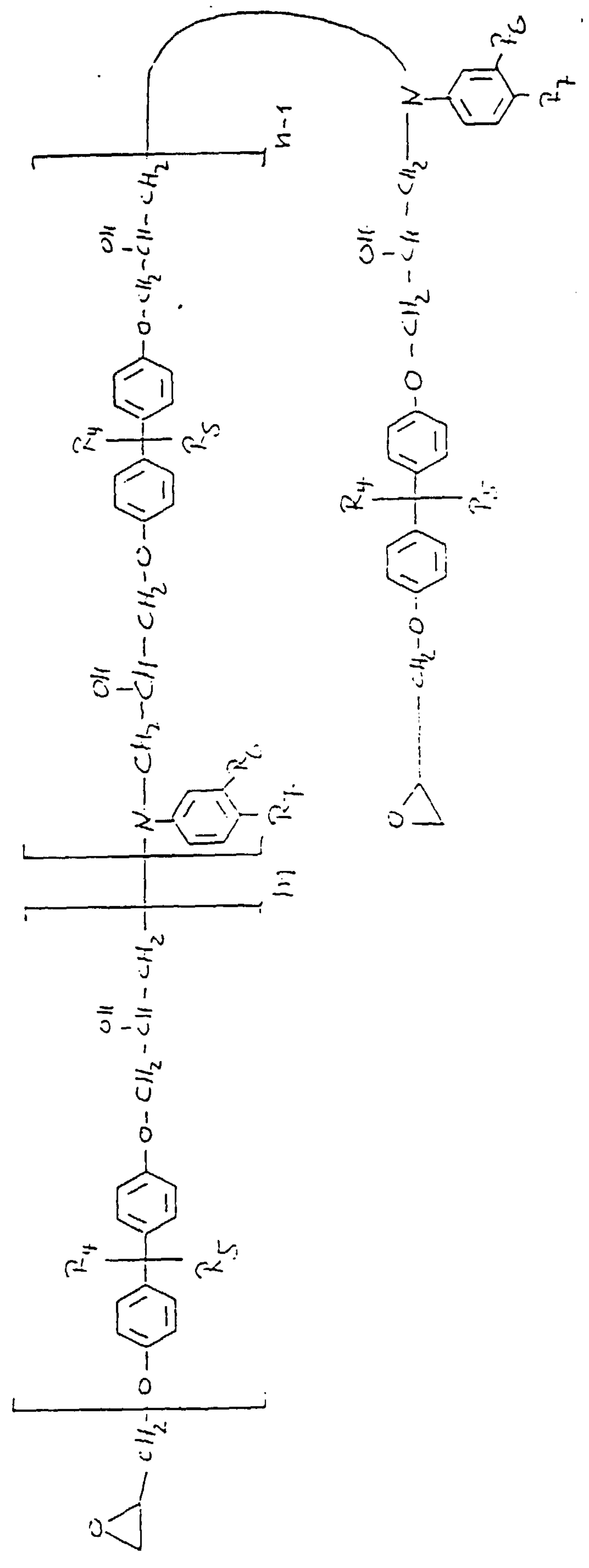

while maintaining a molar ratio (II):(III) of 1.25:1 to 2:1 within a temperature range from 100 to 250°C, wherein $R_1$ to $R_7$ and m are defined as in Claim 1.

**5.** Use of the compounds according to Claims 1 to 3 as a hardening accelerator for ethylenically unsaturated cold hardenable acrylic and polyester resins.

## Revendications

**1.** Composés de formule (I)

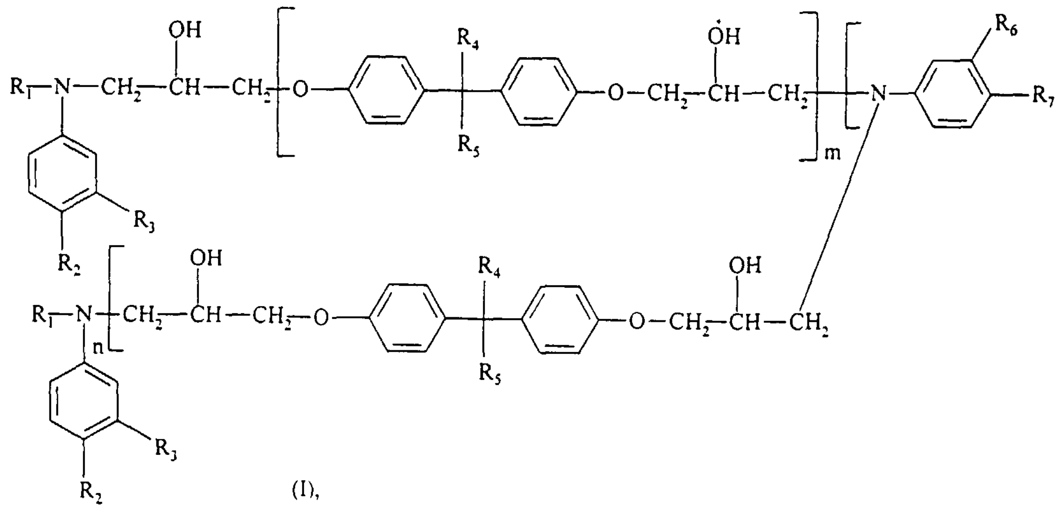

(I),

dans laquelle

| | |
|---|---|
| $R_1$ | représente un groupe alkyle ou cycloalkyle en $C_{1-6}$, |
| $R_2$ et $R_3$, | ayant des significations identiques ou différentes, représentent chacun un atome d'hydrogène, un groupe alkyle ou cycloalkyle en $C_1$-$C_6$ ou un atome d'halogène, |
| $R_4$ et $R_5$ | représentent chacun un groupe méthyle ou bien, ensemble et avec l'atome de carbone reliant les cycles aromatiques, un radical de cyclohexane portant éventuellement des substituants inertes, |
| $R_6$ et $R_7$, | ayant des significations identiques ou différentes, représentent chacun un atome d'hydrogène, un groupe alkyle ou cycloalkyle en $C_1$-$C_6$ ou un atome d'halogène. |
| m | représente un nombre entier ou bien (en moyenne statistique) un nombre fractionnaire allant de 1 à 3 et |
| n | représente un nombre entier ou bien (en moyenne statistique) un nombre fractionnaire allant de 0 à 2, |

sous réserve que, lorsque n = 0, $R_1$ représente un groupe éthyle et $R_2$ un groupe méthyle lorsque $R_3$ représente l'hydrogène et $R_3$ représente un groupe méthyle lorsque $R_2$ représente l'hydrogène.

**2.** Composés selon la revendication 1, caractérisés en ce que l'un des symboles $R_2$ et $R_3$ représente l'hydrogène et l'autre un groupe méthyle.

**3.** Composés selon les revendications 1 et 2, caractérisés en ce que $R_1$ représente un groupe méthyle ou éthyle.

**4.** Procédé de préparation des composés de formule (I) caractérisé en ce que l'on fait réagir une amine de formule (II)

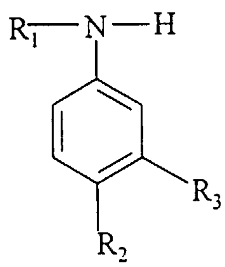

(II)

avec un diépoxyde de formule (III)

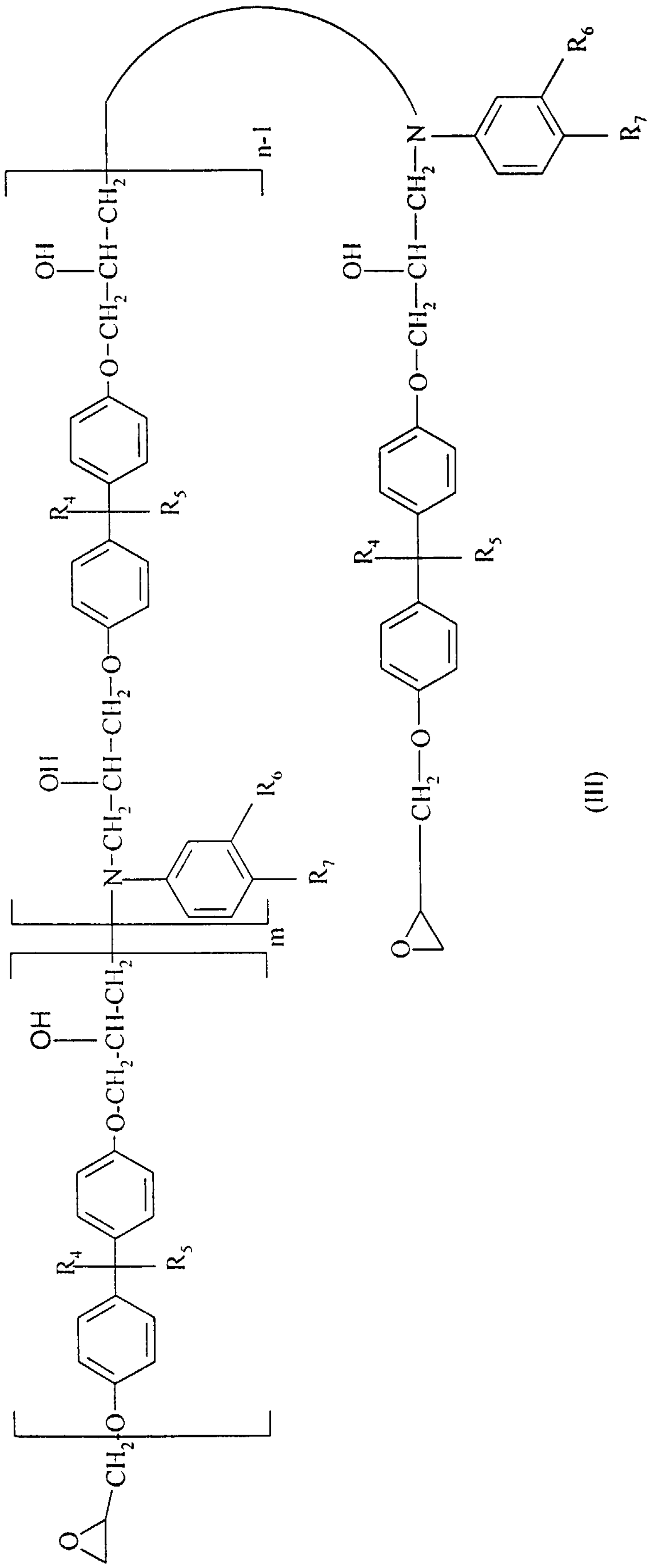

(III)

en respectant un rapport molaire (II)/(III) de 1,25:1 à 2:1, dans l'intervalle de température de 100 à 250°C, les symboles $R_1$ à $R_7$ et m ayant les significations indiquées dans la revendication 1.

**5.** Utilisation des composés selon les revendications 1 à 3, en tant qu'accélérateurs de durcissement pour des résines acryliques et des résines de polyesters à insaturation éthylénique durcissables à froid.